# EUROPEAN PATENT APPLICATION

(11) **EP 1 878 420 A1**
(43) Date of publication of application: **16.01.2008**
(21) Application number: 06745831.5
(22) Date of filing: 28.04.2006
(51) Int. Cl.: A61K 8/39, A61Q 19/00

(54) **SKIN REGENERATION PROMOTER**

(30) Priority: 28.04.2005 JP 2005130971
(71) Applicant: Japan Science and Technology Agency, Kawaguchi-shi, Saitama 332-0012 (JP); Nanoegg Research Laboratories, Inc., Kawasaki-shi Kanagawa 216-8512 (JP)
(72) Inventor: YAMAGUCHI, Yoko, Ashigara Kami-gun, Kanagawa 2580022 (JP); IGARASHI, Rie, Kawasaki-shi, Kanagawa 2140036 (JP)
(74) Representative: Walcher, Armin
(86) International application number: PCT/JP2006/308972
(87) International publication number: WO 2006/118245

(57) **Abstract**

An object of the present invention is to provide a novel dermal regeneration enhancer. In accordance with the present invention, there is provided a dermal regeneration enhancer as a novel pharmaceutical use of lyotropic liquid crystal which has been utilized as a basic material for pharmaceutical preparations for external application and for cosmetics, and the dermal regeneration enhancer of the present invention achieves an excellent suppressive effect to aging of the skin, generation of spots, etc.

## Description

### Technical Field

The present invention relates to a novel dermal regeneration enhancer which achieves its effect by means of external application.

### Background Art

Skin comprises epidermis and dermis, and the epidermis is layered in the order of basal layer, prickle layer, granular layer and horny layer from the inner side. In keratinized cells (keratinocytes) constituting the epidermis, metabolism (turnover) where basal cells produced by cell division are differentiated in the order of prickle cells, granular cells and horny cells and detached from the surface as keratin pieces is repeated (its period is said to be 28 days in the healthy skin). When the turnover does not take place normally, then various troubles happen and aging phenomenon of the skin, a phenomenon where melanin pigment produced in pigment cells in epidermis is not discharged but remains as spots (pigment deposition), etc. are becoming noticeable. Accordingly, it is very important in terms of both medicine and beauty that turnover of the epidermis takes place normally so that the skin is always regenerated.

As a method which has been known already where differentiation and growth of keratinocytes are enhanced whereby regeneration of the skin is enhanced, there is a method where an agent for external application containing a substance which has a differentiation and growth-enhancing action for keratinocytes such as retinoic acid (vitamin A acid) is applied on the skin surface. However, it is not easy that an effective ingredient is permeated into the body via the skin constituting the primary barrier of the living body and its bioavailability (amount of the drug absorbed with a blood flow) is inherently low. Accordingly, in order to achieve the improvement of bioavailability of effective ingredients, it has been conducted that dipropylene glycol, hexylene glycol, isoparaffin, sodium laurylsulfate, an ethylene oxide adduct of lauryl alcohol, polyethylene glycol fatty acid ester, polyoxyethylene sorbitan fatty acid ester, propyl carbonate, sodium pyrrolidonecarboxylate, urea, lactic acid, sodium lactate, lecithin, dimethyl sulfoxide, pyrrolidonecarboxylate, nicotinate, N-methylproline ester, cholesteryl oleate, amine oxide or the like is compounded with preparations for external application as a transdermal absorption enhancer.

Up to now, the present inventors have energetically carried out research and development for dermal regeneration enhancers and, as a result, they have found that, when retinoic acid is included into capsules of a nanometer level (nano-particles) followed by applying to the skin surface, retinoic acid is able to be transdermally absorbed in efficient and sustained-releasing manner without compounding of the transdermal absorption enhancers (Non-Patent Document 1 and Non-Patent Document 2).
Non-Patent Document 1: Yoko Yamaguchi, "Novel Nano-Technology for Transdermal Delivery", Bio Venture, vol. 4, no. 6, pages 62 to 64, 2004
Non-Patent Document 2: Y. Yamaguchi, T. Nagasawa, N. Nakamura, M. Takenaga, M. Mizoguchi, S. Kawai, Y. Mizushima and R. Igarashi, "Successful Treatment of Photo-Damaged Skin of Nano-Scale atRA Particles Using a Novel Transdermal Delivery", 104, 29 to 40, 2005.

### Disclosure of the Invention

### Problems that the Invention is to Solve

In the above-mentioned dermal regeneration enhancer where nano-particle including retinoic acid therein is an effective ingredient, its effect is high and the irritation of retinoic acid to the skin is little whereby its clinical application is expected, but investigation of far better dermal regeneration enhancers is still meaningful.
Accordingly, an object of the present invention is to provide a novel dermal regeneration enhancer.

### Means for Solving the Problems

During the course of investigation on basic materials of external application for skin to be compounded with the above-mentioned nano-particles including retinoic acid therein, the present inventors have found that lyotropic liquid crystal itself which is aimed to utilize as a basic material has a dermal regeneration enhancing action. Although lyotropic liquid crystal has been already known as a basic material for pharmaceutical preparations for external application and for cosmetics (refer, for example, to Japanese Patent Nos. 2,547,151 and 3,459,253), there has been no document which mentions or suggests that lyotropic liquid crystal has an enhancing action for dermal regeneration.

The dermal regeneration enhancer of the present invention achieved on the basis of the above finding is characterized in that lyotropic liquid crystal is an effective ingredient as mentioned in claim 1.
The dermal regeneration enhancer mentioned in claim 2 is characterized in that, in the dermal regeneration enhancer according to claim 1, the lyotropic liquid crystal contains 5% by weight to 80% by weight of a surfactant and 5% by weight to 80% by weight of water.
The dermal regeneration enhancer mentioned in claim 3 is characterized in that, in the dermal regeneration enhancer according to claim 2, the surfactant is a nonionic surfactant and/or lecithin.
The dermal regeneration enhancer mentioned in claim 4 is characterized in that, in the dermal regeneration enhancer according to claim 3, the nonionic surfactant is at least one member selected from the group consisting of polyoxyethylene alkyl ether, polyoxyethylene sorbitan fatty acid ester and polyoxyethylene hydrogenated castor oil.
The dermal regeneration enhancer mentioned in claim 5 is characterized in that, in the dermal regeneration enhancer according to claim 2, the lyotropic liquid crystal further contains 1% by weight to 80% by weight of oil.
The dermal regeneration enhancer mentioned in claim 6 is characterized in that, in the dermal regeneration enhancer according to claim 5, the oil is squalane.
The dermal regeneration enhancer mentioned in claim 7 is characterized in that, in the dermal regeneration enhancer according to claim 2, the lyotropic liquid crystal further contains 1% by weight to 55% by weight of a polyhydric alcohol.
The dermal regeneration enhancer mentioned in claim 8 is characterized in that, in the dermal regeneration enhancer according to claim 7, the polyhydric alcohol is glycerol.
The dermal regeneration enhancer mentioned in claim 9 is characterized in that, in the dermal regeneration enhancer according to claim 2, the lyotropic liquid crystal further contains 0.01% by weight to 10% by weight of an auxiliary surfactant.
The dermal regeneration enhancer mentioned in claim 10 is characterized in that, in the dermal regeneration enhancer according to claim 9, the auxiliary surfactant is cholesterol.
The dermal regeneration enhancer mentioned in claim 11 is characterized in that, in the dermal regeneration enhancer according to claim 1, the lyotropic liquid crystal is compounded with a substance having an enhancing action for differentiation and growth of keratinocytes and/or a substance having a suppressive action to melanin pigment production.
The dermal regeneration enhancer mentioned in claim 12 is characterized in that, in the dermal regeneration enhancer according to claim 11, the substance having an enhancing action for differentiation and growth of keratinocytes is at least one member selected from the group consisting of retinal, 3-dehydroretinal, retinoic acid, 3-dehydroretinoic acid, substances similar to retinoic acid, retinol, retinol fatty acid ester and 3-dehydroretinol fatty acid ester.
The dermal regeneration enhancer mentioned in claim 13 is characterized in that, in the dermal regeneration enhancer according to claim 11, the substance having a suppressive action to melanin pigment production is at least one member selected from the group consisting of ascorbic acid glucoside, arbutin and superoxide dismutase.
The dermal regeneration enhancer mentioned in claim 14 is characterized in that, in the dermal regeneration enhancer according to claim 11, the substance having an enhancing action for differentiation and growth of keratinocytes and/or the substance having a suppressive action to melanin pigment production are/is compounded in a form of being included in the inside of fine particles of inorganic acid salt with divalent metal.

### Advantages of the Invention

In accordance with the present invention, there is provided a dermal regeneration enhancer as a novel pharmaceutical use of lyotropic liquid crystal which has been utilized as a basic material for pharmaceutical preparations for external application and for cosmetics, and the dermal regeneration enhancer of the present invention achieves an excellent suppressive effect to aging of the skin, generation of spots, etc. Incidentally, in the present invention, *"*skin*"* mainly means epidermis but it does not exclude mucous membrane.

### Brief Description of the Drawings

Fig. 1 is a cross-sectional picture of the skin to which the lyotropic liquid crystal compounded with no retinoic acid was applied as mentioned in (A) of Example 1.
Fig. 2 is a cross-sectional picture of the skin to which the lyotropic liquid crystal compounded with the nano-particles including retinoic acid therein was applied as mentioned in (B) of Example 1.
Fig. 3 is a cross-sectional picture of the skin to which nothing was applied as mentioned in (C) of Example 1.
Fig. 4 is a graph which shows changes in the production amounts of HB-EGF when each of the four kinds of lyotropic liquid crystals was applied and the production amount of HB-EGF of the skin to which nothing was applied as mentioned in Example 2.
Fig. 5 shows cross-sectional pictures of the skin to which each of the four kinds of samples which are lotions containing the lyotropic liquid crystal compounded with the three kinds of ratios and the lotion base only was applied as mentioned in Example 3.
Fig. 6 shows cross-sectional pictures of the skin to which each of the four kinds of samples of the lyotropic liquid crystal and the constituting components thereof - surfactant, oil and polyhydric alcohol - was applied and a cross-sectional picture of the skin to which nothing was applied as mentioned in Example 4.
Fig. 7 shows cross-sectional pictures of the skin to which each of the four kinds of samples which are lotions containing the lyotropic liquid crystal compounded with the three kinds of ratios and the lotion base only was applied as mentioned in Example 5.
Fig. 8 is a graph which shows the changes in viscoelasticity of the skin with the passage of time when the lyotropic liquid crystal was applied as mentioned in Example 6.
Fig. 9 shows pictures of the surfaces of horny cells of the skin to which each of the two kinds of samples where one is a lotion containing the lyotropic liquid crystal and another is a lotion base only was applied as mentioned in Example 7.
Fig. 10 is a graph which shows the changes of water amount in horny layer with the passage of time as mentioned in Example 7.
Fig. 11 is a cross-sectional picture of the skin to which the lyotropic liquid crystal of the formulation 1 as mentioned in Example 9 was applied.
Fig. 12 is a cross-sectional picture of the skin to which the lyotropic liquid crystal of the formulation 2 as mentioned in Example 9 was applied.
Fig. 13 is a cross-sectional picture of the skin to which nothing was applied as mentioned in Example 9.

### Best Mode for Carrying Out the Invention

The dermal regeneration enhancer according to the present invention is characterized in that lyotropic liquid crystal is an effective ingredient. The lyotropic liquid crystal in accordance with the present invention means such a thing that, in a system where surfactant (amphipathic molecule having a hydrophilic part and a hydrophobic (lipophilic) part in a molecule) and water are coexisting, a liquid crystal state (a state where a predetermined regularity in molecular orientation is maintained as if in the case of crystal while fluidity is still available as if in the case of liquid) is formed depending upon the mixing ratio of both parts and upon temperature. Principally, it is able to be understood that, in lyotropic liquid crystal, when water is added, within a predetermined temperature range, to a surfactant in a solid state having a crystal structure where hydrophobic parts (hydrophobic groups such as alkyl group) are faced each other, said parts lose regularity due to thermal movement resulting in a liquid state and then the hydrophilic parts act each other due to hydrogen bond to maintain for a long period whereby an associated structure (such as hexagonal structure and lamella structure) is resulted (refer, if necessary, to Toshiyuki Suzuki, "Liquid Crystal", vol. 2, pages 194 to 201, 1998).

With regard to the surfactant which is a constituting component of the lyotropic liquid crystal, there is no particular limitation so far as it is able to form a liquid crystal state (a periodical structure where the interplanar spacing is 10 nm to 800 nm is particularly preferred) in a system coexisting with water depending upon the mixing ratio with water and upon temperature. Thus, it may be a surfactant of any of the types of nonionic type, anionic type, cationic type and amphoteric type and may also be a surfactant derived from nature such as lecithin (for example, egg yolk lecithin and soybean lecithin) and saponin. A single surfactant maybe used solely or plural kinds thereof may be mixed and used.

Examples of the nonionic surfactant are polyoxyethylene alkyl ether, polyoxyethylene alkyl phenol ether, alkyl glucoside, polyoxyethylene fatty acid ester, sucrose fatty acid ester, sorbitan fatty acid ester, polyoxyethylene sorbitan fatty acid ester, fatty acid alkanolamide and polyoxyethylene hydrogenated castor oil. Examples of the anionic surfactant are soap (sodium salt, potassium salt, etc. of fatty acid), alkylbenzenesulfonate (such as sodium salt), higher alcohol sulfate salt (such as sodium salt), polyoxyethylene alkyl ether sulfate (such as sodium salt), α-sulfofatty acid ester, α-olefin sulfonate (such as sodium salt), monoalkylphosphate salt (such as sodium salt) and alkanesulfonate (such as sodium salt). Examples of the cationic surfactant are alkyl trimethylammonium salt (such as chloride), dialkyl dimethylammonium salt (such as chloride), alkyl dimethylbenzylammonium salt (such as chloride) and amine salt (such as acetate salt and hydrochloride salt). Examples of the amphoteric surfactant are alkylamino fatty acid salt (such as sodium salt), alkylbetaine and alkylamine oxide. Rate of the surfactant in the lyotropic liquid crystal is preferably 5% by weight to 80% by weight, more preferably 7% by weight to 70% by weight and, still more preferably, 10% by weight to 65% by weight. HLB value of the surfactant is preferably not less than 8, more preferably not less than 10 and, still more preferably, not less than 12.

With regard to water which is a constituting component of the lyotropic liquid crystal, distilled water or the like may be used. Water used therefor may contain organic solvent which is miscible with water such as ethanol and isopropanol. Rate of water in the lyotropic liquid crystal is preferably 5% by weight to 80% by weight, more preferably 10% by weight to 60% by weight and, still more preferably, 13% by weight to 50% by weight.

The lyotropic liquid crystal may further contain oil besides the surfactant and water. When oil is contained therein, the liquid crystal structure becomes similar to a lamella structure formed by the intercellular lipid in a horny layer and, upon application to the skin surface, a phase transfer of the intercellular lipid structure is apt to happen and, as a result, an excellent enhancing action for dermal regeneration is achieved. Examples of the oil are vegetable oil such as wheat germ oil, corn oil, sunflower oil and castor oil; silicone oil; ester oil such as isopropyl myristate, glyceryl trioctanoate, diethylene glycol monopropylene pentaerythritol ether and pentaerythrityl tetraoctanoate; squalane; squalene; liquid paraffin; and polybutene. A single oil may be used solely or plural kinds thereof may be mixed and used. Rate of the oil in the lyotropic liquid crystal is preferably 1% by weight to 80% by weight, more preferably 5% by weight to 70% by weight and, still more preferably, 10% by weight to 65% by weight.

The lyotropic liquid crystal may further contain a polyhydric alcohol. When a polyhydric alcohol is contained therein, it is possible to attempt for making the formation of liquid crystal structure easy (expansion of phase region) and for making it stable. Examples of the polyhydric alcohol are polyalkylene glycol (such as polyethylene glycol and polyalkylene glycol), glycerol, propylene glycol, 1,3-propanediol, 2-butene-1,4-diol, pentane-1,5-diol, 2,2-dimethylpropane-1,3-diol, 3-methylpentane-1,5-diol, pentane-1,2-diol, 2,2,4-trimethylpentane-1,3-diol, 2-methylpropane-1,3-diol, hexylene glycol, 1,3-butylene glycol, dipropylene glycol, diethylene glycol and triethylene glycol. A single polyhydric alcohol may be used solely or plural kinds thereof may be mixed and used. Rate of the polyhydric alcohol in the lyotropic liquid crystal is preferably 1% by weight to 55% by weight, more preferably 3% by weight to 52% by weight and, still more preferably, 5% by weight to 50% by weight.

The lyotropic liquid crystal may further contain an auxiliary surfactant such as cholesterol. When an auxiliary surfactant is contained therein, reduction of surface membrane curvature is able to be achieved even when various kinds of surfactants are used and, therefore, it is able to attempt for making the formation of liquid crystal structure easy and for making it stable. Rate of the auxiliary surfactant in the lyotropic liquid crystal is preferably 0.01% by weight to 10% by weight.

The lyotropic liquid crystal is able to be prepared by mixing of the surfactant and water which are constituting components thereof in a predetermined ratio at predetermined temperature. If necessary, an operation where the constituting component is temporarily warmed before or after mixing may be carried out.

In the dermal regeneration enhancer of the present invention, the lyotropic liquid crystal may be compounded with a substance having an enhancing action for differentiation and growth of keratinocytes and a substance having a suppressive action to melanin pigment production. As a result of compounding of those substances with the lyotropic liquid crystal, the dermal regeneration enhancer of the present invention achieves far better suppressive effect to aging of the skin, generation of spots, etc. Compounding amount of those substances to the lyotropic liquid crystal in terms of ratio by weight is, for example, from 0.01% to 50%. Examples of the substance having an enhancing action for differentiation and growth of keratinocytes are retinal, 3-dehydroretinal, retinoic acid, 3-dehydroretinoic acid, substances similar to retinoic acid, retinol, retinol fatty acid ester and 3-dehydroretinol fatty acid ester. Examples of the substance having a suppressive action to melanin pigment production are ascorbic acid glucoside, arbutin and superoxide dismutase. Such a substance itself may be uniformly dispersed in the lyotropic liquid crystal followed by being incorporated among the phases of the liquid crystal structure so that it is compounded, or it may be included in the inside of fine particles of inorganic acid salt with divalent metal such as fine particles where diameter is 100 nm to 1,000 nm comprising calcium carbonate, magnesium carbonate, zinc carbonate, calcium phosphate, magnesium phosphate and zinc phosphate (with regard to a method therefor, refer, if necessary, to WO 02/096396) and the fine particles (nano-particles) into which such a substance is included are uniformly dispersed in the lyotropic liquid crystal followed by being incorporated among the phases of the liquid crystal structure so that they are compounded. In addition, a divalent metal ion and a counterion thereof are adsorbed on the surface (surface membrane) of the lyotropic liquid crystal so as to enhance the viscoelasticity of the membrane, whereby the physical and chemical stability of the substance incorporated among the phases is able to be improved.

In the dermal regeneration enhancer of the present invention, the lyotropic liquid crystal which has been utilized as a basic material for pharmaceutical preparations for external application and for cosmetics is an effective ingredient. Therefore, it may be directly applied to the skin surface as a preparation for external application or may be applied to the skin surface after dispersing in an ointment base, a cream base or a lotion base. It goes without saying that, in making into the preparations, known components such as antiseptic, moisturizer or antioxidant is appropriately added thereto.

### Examples

### Example 1:

### (A: Example)

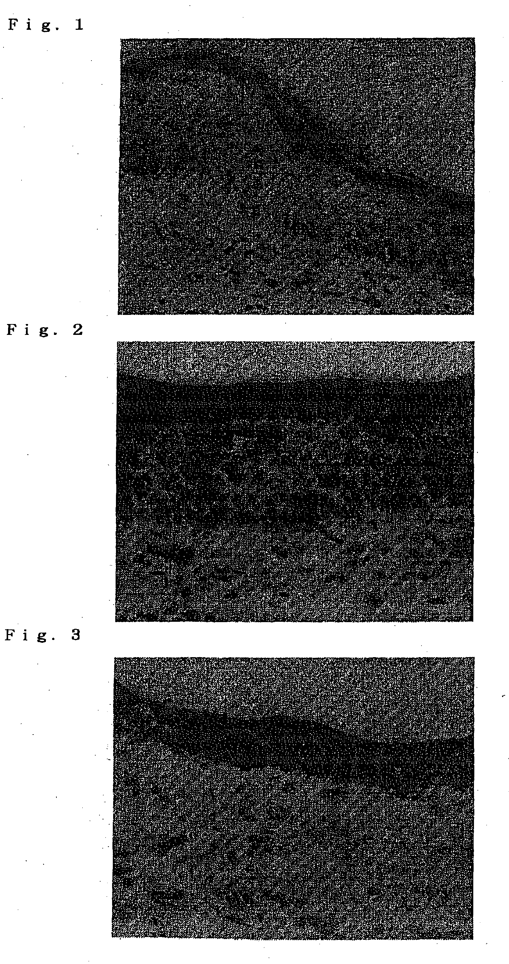

31 mL of glycerol (a polyhydric alcohol) was added to a beaker in which 17 mL of distilled water was placed so that it was uniformly dissolved. Then 28 mL of Emulgen 2020G-HA (polyoxyethylene octyl dodecyl ether) which is a trade name of a nonionic surfactant manufactured by Kao was added thereto and uniformly dispersed therein. Since viscosity of the solution increased at that time, such a phenomenon was used as a yardstick for the uniform dispersion of each of the materials. After that, 20 mL of squalane (an oil) was added to uniformly mix therewith, then 10 mL of squalene was further added and the mixture was stirred for about 5 minutes. More 5 mL of squalane was added and the mixture was stirred, whereupon viscosity of the solution gradually rose and it was instantly gelled. This phenomenon was used as a yardstick for the formation of the liquid crystal. After that, stirring was still continued for several minutes to give lyotropic liquid crystal (comprising 28.0% by weight of surfactant, 16.0% by weight of water, 25.0% by weight of oil and 31.0% by weight of polyhydric alcohol). Incidentally, all of the above operations were carried out under shielding the light and the nonionic surfactant was used after being melted at about 60°C (hereinafter, that is also the same).
Back of colored guinea pigs having melanin pigment-producing cells (Weiser Maples; six weeks age; male) was shaved, the shaved part was washed with lukewarm water and 30 mg of the above lyotropic liquid crystal was applied to an area of 2 cm × 5 cm thereof. After 2 days from the application date under irradiation with any of UVA, UVB and UVA + UVB, skin of the part to which the lyotropic liquid crystal was applied was collected and the slice was fixed with formalin, embedded in paraffin and stained by a Fontana-Masson method where melanin pigment was stained out in black to evaluate the dermal regeneration enhancing action. The cross-sectional picture of the skin is shown in Fig. 1.

### (B: Example)

140 mg of retinoic acid (all-trans substance), 400 µL of ethanol and 560 µL of a 1N aqueous solution of sodium hydroxide were placed in a beaker so that retinoic acid was uniformly dissolved. Then 5 mL of glycerol and 2 mL of Emulgen 2020G-HA were added thereto followed by stirring for about 10 minutes. Then 17.72 mL of distilled water was added thereto and the mixture was stirred for about 10 minutes to give a mixed micelle of retinoic acid and the nonionic surfactant. After that, 46.5 µL of a 5M aqueous solution of magnesium chloride was added thereto followed by stirring for about 1 hour. Finally, 46.5 µL of a 1M aqueous solution of sodium carbonate was added thereto and the mixture was stirred for about 1 hour to give nano-particles in which retinoic acid was included in a thin film of magnesium carbonate where the diameter was 100 nm to 1000 nm. The nano-particles were compounded with the lyotropic liquid crystal prepared in (A) so as to make the compounding amount of retinoic acid 0.1% by weight to the lyotropic liquid crystal to give the lyotropic liquid crystal where the nano-particles in which retinoic acid was included were uniformly dispersed without degradation. The dermal regeneration enhancing action of the lyotropic liquid crystal compounded with the nano-particles in which retinoic acid was included was evaluated by the method mentioned in the above (A). A cross-sectional picture of the skin is shown in Fig. 2.

### (C: Control)

Skin to which nothing was applied was collected, the slice was fixed with formalin, embedded in paraffin and stained by a Fontana-Masson method where melanin pigment was stained out in black. A cross-sectional picture of the skin is shown in Fig. 3.

As will be apparent from Fig. 1 to Fig. 3, when the lyotropic liquid crystal of (A) or (B) was applied, thickening of the epidermis was significant as compared with the control (C) regardless of presence or absence of retinoic acid, and excellent dermal regeneration enhancing action was achieved. When the lyotropic liquid crystal of (A) was applied, amount of melanin pigment was greatly small as compared with the control (C) (being judged from the fact that black spots and areas were little).

### Example 2:

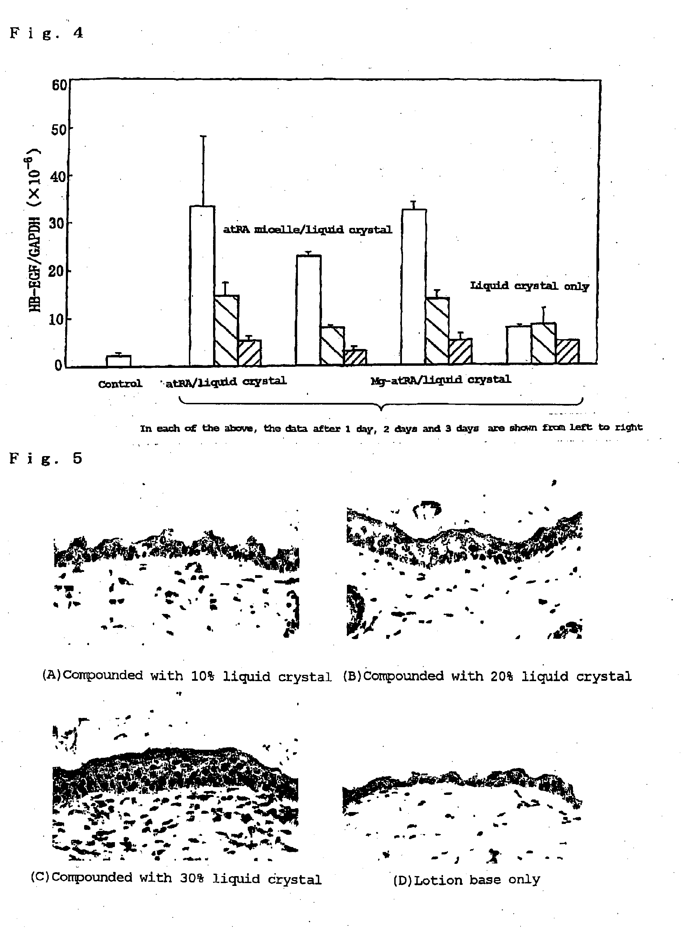

Back of ddY mice (seven weeks age, male) was shaved, the shaved part was washed with lukewarm water and each 30 mg of the four kinds of lyotropic liquid crystal of the following (a) to (d) was applied to an area of 1.5 cm × 1.5 cm thereof. Changes in the production amount of HB-EGF (heparin-binding EGF-like growth factor) playing a role of dermal regeneration function after 1 day, 2 days and 3 days from the application date were measured (refer, if necessary, to Non-Patent Document 2 for the details of the measuring means) and dermal regeneration enhancing action of each of them was evaluated. The result is shown in Fig. 4 together with the production amount of HB-EGF of the skin to which nothing was applied. As will be apparent from Fig. 4, an increasing action for HB-EGF production is noted for the lyotropic liquid crystal itself and said action is enhanced by compounding with retinoic acid independently of its compounding form. Incidentally, the lyotropic liquid crystals of (a) to (c) have an excellent stability for retinoic acid upon preservation and, even after 80 days from the preparation, 95% or more retinoic acid still remained.
(a) Lyotropic liquid crystal compounded with the nano-particles in which retinoic acid was included as mentioned in (B) of Example 1 (Mg-atRA/liquid crystal)
(b) Lyotropic liquid crystal compounded with the mixed micelle of retinoic acid and the nonionic surfactant obtained in the preparation of the nano-particles in which retinoic acid was included as mentioned in (B) of Example 1 so as to make the compounding amount of retinoic acid to the lyotropic liquid crystal 0.1% by weight (atRA micelle/liquid crystal)
(c) Lyotropic liquid crystal where retinoic acid itself was compounded therein so as to make the compounding amount of retinoic acid to the lyotropic liquid crystal 0.1% by weight (atRA/liquid crystal)
(d) Lyotropic liquid crystal where no retinoic acid was compounded as mentioned in (A) of Example 1 (liquid crystal only)

### Example 3:

The four kinds of samples which are lotions prepared by compounding 10%, 20% and 30% (by weight) of the lyotropic liquid crystal of (A) of Example 1 with the home-made lotion base (milky liquid) and a lotion base only were applied for consecutive four days at the rate of 13.5 mg/cm² each to the part where back of ddY mice (five weeks age, male) was shaved and washed with lukewarm water. Then the skin to which the sample was applied was collected, the slice was fixed with formalin, embedded in paraffin and stained with hyaluronic acid (colloidal iron staining) to evaluate the dermal regeneration enhancing action. Cross-sectional pictures of the skin to which the samples were applied are shown in Fig. 5. As will be apparent from Fig. 5, degree of thickness of the epidermis was dependent upon the compounding amount of the lyotropic liquid crystal.

### Example 4:

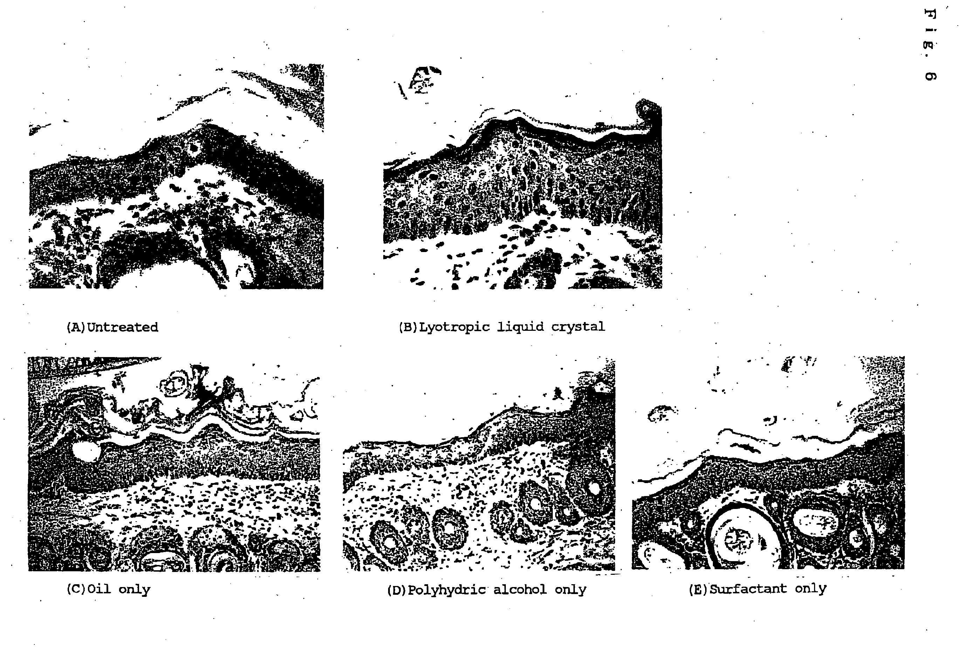

Back of colored guinea pigs having melanin pigment-producing cells (Weiser Maples, five weeks age, male) was shaved, the shaved part was washed with lukewarm water, each 30 mg of the four kinds of samples of the lyotropic crystal of (A) of Example 1 and the constituting components thereof - surfactant, oil and polyhydric alcohol - was applied to an area of 2 cm × 2 cm thereof and the dermal regeneration enhancing action was evaluated by the method mentioned in (A) of Example 1. Cross-sectional pictures of the skin to which each of the samples was applied are shown in Fig. 6 together with the cross-sectional picture of the skin to which nothing was applied. As will be apparent from Fig. 6, no dermal regeneration enhancing action was noted when each of the constituting components of the lyotropic liquid crystal was solely applied, and the dermal regeneration enhancing action was noted in the lyotropic liquid crystal only.

### Example 5:

Dermal regeneration enhancing action of the four kinds of samples was evaluated by the same manner as in Example 3 except that staining with Ki-67 (a marker showing that cells are in a growing state) was conducted instead of staining with hyaluronic acid. Cross-sectional pictures of the skin to which each of the samples was applied are shown in Fig. 7. As will be apparent from Fig. 7, cells in a growing state significantly increased in the skin to which a lotion prepared by compounding with the lyotropic liquid crystals was applied, and it was suggested that, in the dermal regeneration enhancing action of the lyotropic liquid crystal, a growth enhancing action to basal cells is one of the causes therefor.

### Example 6:

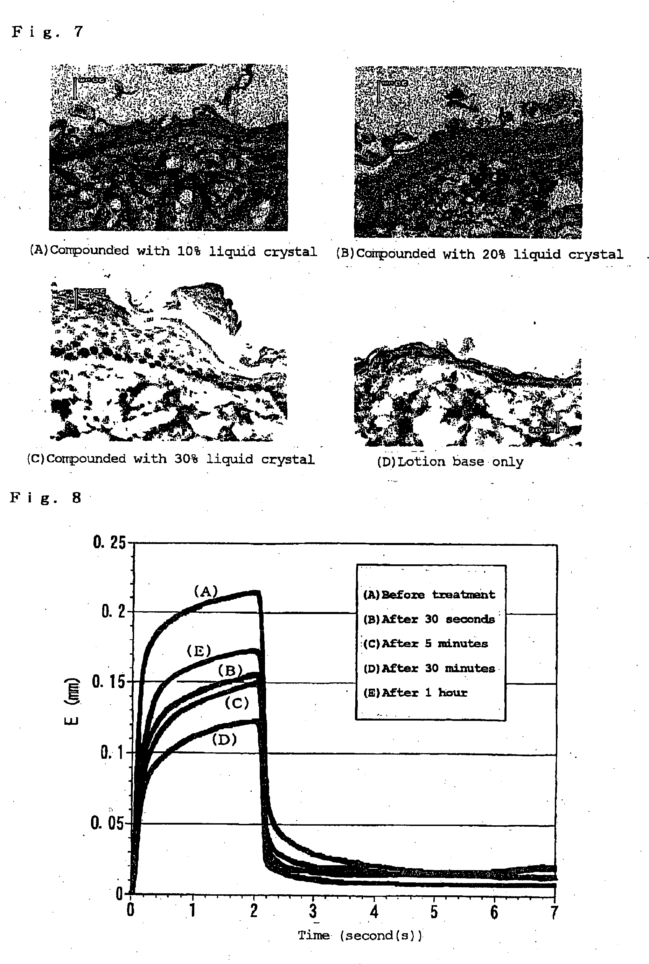

Before the application and 30 seconds, 5 minutes, 30 minutes and 1 hour after the application of 10 mg/cm² of the lyotropic liquid crystal of (A) of Example 1 to the cheek of human (female), the skin of said part was sucked at negative pressure (400 mba) using a cutometer and then instantly released, and the tensile strength and returning degree thereby were measured whereby viscoelasticity of the skin was evaluated. The result is shown in Fig. 8. As will be apparent from Fig. 8, when the lyotropic liquid crystal of (A) of Example 1 was applied, viscoelasticity of the skin lowered with the passage of time until 30 minutes after the application but it increased after 1 hour and was in the same degree until after 5 hours (not shown). The above result is thought to be due to the fact that softening of the skin took place as a result of changes in the structure of the horny layer.

### Example 7:

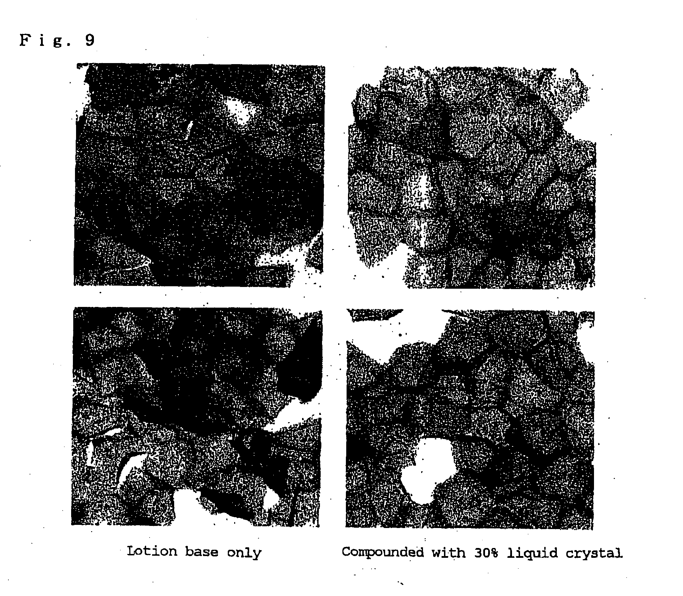

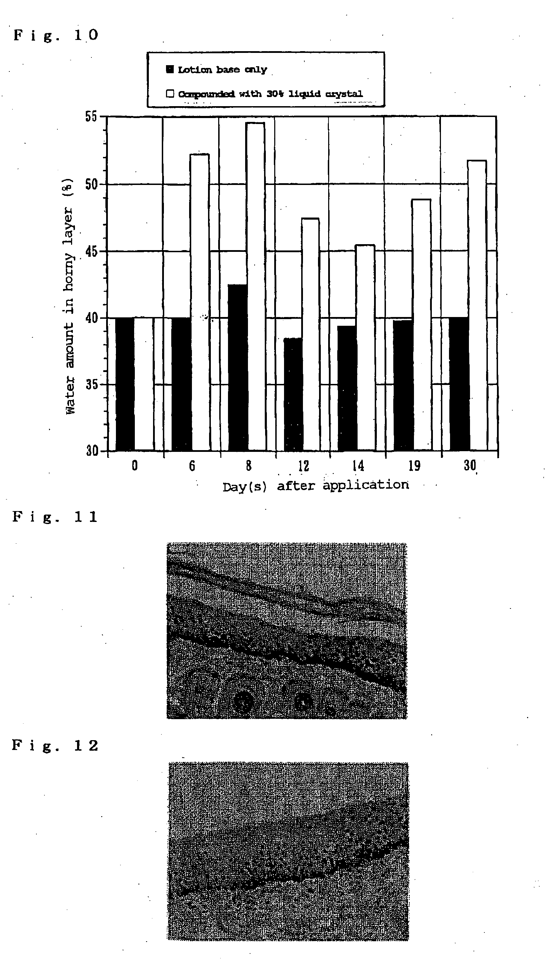

Each of the two kinds of samples where one was a lotion prepared by compounding 30% (by weight) of the lyotropic liquid crystal of (A) of Example 1 with a home-made lotion base (milky liquid) and another was a lotion base only was applied in an appropriate amount to the cheek of human (female) for one month and then horny cells of the skin to be applied were transferred by a tape strip method and stained with a mixed solution of Gentiana Violet and Brilliant Green. Pictures of the surface of horny cells of the skin to which each of the samples was applied are shown in Fig. 9. As will be apparent from Fig. 9, layering of horny cells is less in the case where the lotion prepared by compounding with the lyotropic liquid crystal was applied as compared with the case where the lotion base only was applied, and it was suggested that differentiation of horny cells normally proceeded and turnover of the epidermis was enhanced. Fig. 10 shows the changes, with the passage of time, of water amount in horny layer of the skin to which each of the two kinds of samples was applied as measured by a cutometer. As will be apparent from Fig. 10, as compared with the case where the lotion base only was applied, water amount in horny layer significantly increased when the lotion prepared by compounding with the lyotropic liquid crystal was applied. The above result was thought to be due to the fact that, as a result of the dermal regeneration enhancing action of the lyotropic liquid crystal, production amount of hyaluronic acid in the epidermis layer increased.

### Example 8:

An appropriate amount of each of the two kinds of samples where one is a lotion prepared by compounding with 30% (by weight) of the lyotropic liquid crystal of (A) of Example 1 and also with 2% (by weight) of ascorbic acid glucoside having a whitening effect and an suppressive effect to melanin pigment production with a commercially available lotion base and another is a lotion prepared by compounding with 2% (by weight) of ascorbic acid glucoside only was applied to the cheek of human (female) for two months and the appearances of the skins to which each of the samples was applied were compared. The result was that, as compared with the case where the lotion prepared by compounding only with ascorbic acid glucoside was applied, lightness of the skin was enhanced when the lotion prepared by compounding with the lyotropic liquid crystal and ascorbic acid glucoside was applied. To be more specific, when the changes in lightness of the skin was evaluated in terms of L* value, there was almost no change when the lotion prepared by compounding only with ascorbic acid glucoside was applied where the value was 57.43 before the application while, after the application, it was 56.48. On the other hand, when the lotion prepared by compounding with the lyotropic liquid crystal and ascorbic acid glucoside was applied, it was 57.59 before the application and increased to 60.34 after the application (it was judged that the more the L* value, the more the whiteness). The above result is thought to be due to the fact that turnover of the epidermis was enhanced by the dermal regeneration enhancing action of the lyotropic liquid crystal and also that phase transfer of the intercellular lipid structure of horny layer took place by the application of the lyotropic liquid crystal to the skin surface whereby transdermal absorption of ascorbic acid glucoside was enhanced and spots were removed.

### Example 9:

Lyotropic liquid crystal comprising each of the four kinds of formulations (unit: % by weight) mentioned in Table 1 was prepared by mixing the constituting components. To be more specific, all components except distilled water were mixed and heated at about 60°C so that soybean lecithin, cholesterol and POE (60) hydrogenated castor oil were melted therein, and predetermined amount of distilled water was added thereto followed by stirring whereupon the product was prepared.

**[Table 1]**

| | Formulation 1 | Formulation 2 | Formulation 3 | Formulation 4 |
|---|---|---|---|---|
| Squalane | 16.819 | 17.948 | 25.948 | 23.000 |
| Soybean Lecithin | 8.931 | 9.570 | 9.570 | 10.000 |
| Cholesterol | 4.466 | 3.828 | 3.828 | 3.333 |
| POE (60) Hydrogenated Castor Oil | 15.026 | 5.200 | 5.200 | 3.500 |
| Glycerol | 38.897 | 44.247 | 41.247 | 45.967 |
| Distilled Water | 15.860 | 19.207 | 14.207 | 14.200 |
| Total | 100.000 | 100.000 | 100.000 | 100.000 |

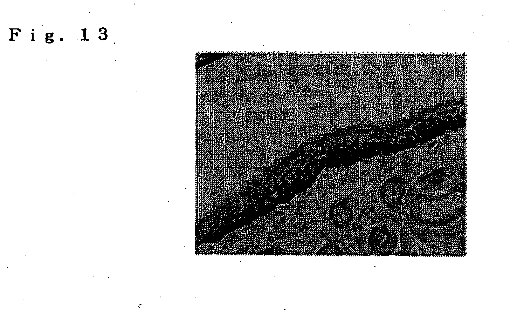

Back of colored guinea pigs having melanin pigment-producing cells (Weiser Maples, five weeks age, male) was shaved, the shaved part was washed with lukewarm water, each 30 mg of the lyotropic liquid crystal of the formulation 1 and that of the formulation 2 was applied to an area of 2 cm × 2 cm thereof for consecutive ten days under irradiation with any of UVA, UVB and UVA + UVB and the dermal regeneration enhancing action was evaluated by the method mentioned in (A) of Example 1. A cross-sectional picture of the skin to which the lyotropic liquid crystal of the formulation 1 was applied is shown in Fig. 11, a cross-sectional picture of the skin to which the lyotropic liquid crystal of the formulation 2 was applied is shown in Fig. 12 and a cross-sectional picture of the skin to which nothing was applied is shown in Fig. 13. As will be apparent from Fig. 11 to Fig. 13, thickening of the epidermis is significant when the lyotropic liquid crystal of the formulation 1 and that of the formulation 2 was applied as compared with the control, a dermal regeneration enhancing action was excellent and amount of melanin pigment was less than that in the case of the control.

### Preparation Example 1:

A commercially available antiseptic was added to the lyotropic liquid crystal of the formulation 1 of Example 9 to prepare a product.

### Preparation Example 2:

The lyotropic liquid crystal of the formulation 2 of Example 9 was compounded with a home-made lotion base (milky liquid) and then a commercially available antiseptic was added thereto to prepare a lotion. The lotion base was prepared by mixing of soybean lecithin, cholesterol, PEG 4000, cyclic silicone, Carbopol (macromolecular gelling agent), Keltrol (macromolecular gelling agent) and distilled water followed by emulsifying.

### Industrial Applicability

The present invention has an industrial applicability in such a respect that there is provided a dermal regeneration enhancer as a novel pharmaceutical use of lyotropic liquid crystal which has been utilized as a basic material for pharmaceutical preparations for external application and for cosmetics.

## Claims

1. A dermal regeneration enhancer, **characterized in that**, lyotropic liquid crystal is an effective ingredient.

2. The dermal regeneration enhancer according to claim 1, wherein said lyotropic liquid crystal contains 5% by weight to 80% by weight of a surfactant and 5% by weight to 80% by weight of water.

3. The dermal regeneration enhancer according to claim 2, wherein said surfactant is a nonionic surfactant and/or lecithin.

4. The dermal regeneration enhancer according to claim 3, wherein said nonionic surfactant is at least one member selected from the group consisting of polyoxyethylene alkyl ether, polyoxyethylene sorbitan fatty acid ester and polyoxyethylene hydrogenated castor oil.

5. The dermal regeneration enhancer according to claim 2, wherein said lyotropic liquid crystal further contains 1% by weight to 80% by weight of oil.

6. The dermal regeneration enhancer according to claim 5, wherein said oil is squalane.

7. The dermal regeneration enhancer according to claim 2, wherein said lyotropic liquid crystal further contains 1% by weight to 55% by weight of a polyhydric alcohol.

8. The dermal regeneration enhancer according to claim 7, wherein said polyhydric alcohol is glycerol.

9. The dermal regeneration enhancer according to claim 2, wherein said lyotropic liquid crystal further contains 0.01% by weight to 10% by weight of an auxiliary surfactant.

10. The dermal regeneration enhancer according to claim 9, wherein said auxiliary surfactant is cholesterol.

11. The dermal regeneration enhancer according to claim 1, wherein said lyotropic liquid crystal is compounded with a substance having an enhancing action for differentiation and growth of keratinocytes and/or a substance having a suppressive action to melanin pigment production.

12. The dermal regeneration enhancer according to claim 11, wherein said substance having an enhancing action for differentiation and growth of keratinocytes is at least one member selected from the group consisting of retinal, 3-dehydroretinal, retinoic acid, 3-dehydroretinoic acid, substances similar to retinoic acid, retinol, retinol fatty acid ester and 3-dehydroretinol fatty acid ester.

13. The dermal regeneration enhancer according to claim 11, wherein said substance having a suppressive action to melanin pigment production is at least one member selected from the group consisting of ascorbic acid glucoside, arbutin and superoxide dismutase.

14. The dermal regeneration enhancer according to claim 11, wherein said substance having an enhancing action for differentiation and growth of keratinocytes and/or the substance having a suppressive action to melanin pigment production are/is compounded in a form of being included in the inside of fine particles of inorganic acid salt with divalent metal.
